# EUROPEAN PATENT SPECIFICATION

(11) **EP 0 960 337 B1**
(45) Date of publication and mention of the grant of the patent: **05.03.2008**
(21) Application number: 98902103.5
(22) Date of filing: 30.01.1998
(51) Int. Cl.: G01N 33/574, G01N 33/573, C12Q 1/37, C12Q 1/68, A61K 39/395

(54) **Cathepsin K and breast cancer**
Cathepsin K und Brustkrebs
Cathepsine K et cancer du sein

(30) Priority: 30.01.1997 GB 9701888
(43) Date of publication of application: 01.12.1999
(73) Proprietor: THE UNIVERSITY OF LIVERPOOL, Liverpool L69 3BX (GB)
(72) Inventor: GALLAGHER, James, Anthony, Crosby Liverpool L23 2UX (GB); LITTLEWOOD-EVANS, Amanda, Jane, CH-4104 Oberwil (CH); BILBE, Graeme, CH-2000 Neuchatel (CH)
(74) Representative: W.P. Thompson & Co.
(86) International application number: PCT/GB1998/000288
(87) International publication number: WO 1998/034117

(56) References cited:
- EP-A- 0 812 916
- WO-A-95/24182
- WO-A-96/13523
- WO-A-97/49668
- VELASCO, G. ET AL.: "Human Cathepsin O; Molecular cloning from a breast carcinoma, production of the active enzyme in escherichia coli, and expression analysis in human tissue." THE JOURNAL OF BIOLOGICAL CHEMISTRY, vol. 269, no. 43, 28 September 1994, pages 27136-17142, XP002065342
- LITTLEWOOD-EVAS, A.J. : "The OSteoclast protease Cathepsin K is expressed in human breast carcinoma." CANCER RESEARCH, vol. 57, 1 December 1997, pages 5386-5390, XP002065343
- HADMAN, M. ET AL.: "IDSOLATION AND CLONING OF jtap-1: A CATHEPSIN LIKE GENE UPREGULATED IN RESPONSE TO V-JUN INDUCED CELL TRANSFORMATION." ONCOGENE, vol. 12, no. 1, 4 January 1994, pages 135-142, XP002065344

## Description

The present invention relates to diagnostic means to detect expression of cathepsin K in breast cells or tissues being indicative for breast cancer and breast cancer metastases, to methods to screen for potential antagonists for cathepsin K capable of inhibiting its effects with respect to breast cancer and related metastasis, as well as to the use of specific substances in the preparation of a composition for use in the treatment of breast cancer and breast cancer metastasis.

Human cathepsin K is the most recently cloned member of the cathepsin family (Inaoka. T., Bilbe. G. et al., Molecular cloning of human cDNA for cathepsin K: Novel cysteine proteinase predominantly expressed in bone; Biochem. Biophys. Res. Commun., 206:89-96; 1995). It is a cysteine protease which was originally isolated from osteoclasts and is believed to play a significant role in the resorption of bone. Cathepsin K has been demonstrated in vitro to be active under acidic conditions, degrading major matrix components such as collagen and osteonectin (Bossard, M.J. et al., Proteolytic activity of human osteoclast cathepsin K, J. Biol. Chem., 271:12517-12524; 1996). Previous studies on the tissue distribution of cathepsin K mRNA revealed that the expression of this protease is specifically and solely localised in osteoclasts. For example, WO 95/24182 discloses cathepsin K and nucleotide sequences coding thereof and provides antibodies, antagonists and inhibitors of said protease alleged to be therapeutically useful to treat bone diseases such as osteoporosis and related bone disorders. Since it has been suggested in previous reports that certain members of the cathepsin family are involved in glomerulonephritis, arthritis and cancer metastasis, WO 95/24182 also refers to the treatment of tumor metastasis. However, this suggestion is solely supported for cathepsin L and B.

In the course of studies investigating the expression of cathepsin K in skeletal tissues, it was unexpectedly noted, that in addition to localising to osteoclasts, the protease was also expressed in breast cancer cells within a bone metastasis. Thus these observations were extended by analyzing the expression of cathepsin K in both primary and metastasised breast tumours and in a number of breast cancer lines.

By PCR and subsequent Southern analysis, twelve out of fourteen breast cancer lines expressed cathepsin K, whereas five breast lines derived from a non-cancerous origin, and thus regarded as possessing a normal or near normal karyotype, were all negative for cathepsin K. Using these techniques it was also possible to detect expression of cathepsin K in samples of primary breast tumours and metastases. Cancer Research, 1997 Vol .57 p. 5386-5390.
From these observations and the fact that cathepsin K degrades major bone matrix components it was concluded that the expression of cathepsin K in breast cancer cells and tissues plays a role in breast cancer metastasis.

### Summary of the invention

According to the present invention, there are provided methods to screen for and evaluate potential antagonists for cathepsin K being capable of inhibiting or at least lowering cathepsin K activity as well as diagnostic means for the detection of expression of cathepsin K in breast cells and tissues being indicative of breast cancer and related metastasis. The present invention further provides use of an antibody or an immunologically active fragment thereof or of antisense nucleotide sequences or of other potential antagonists to cathepsin K activity identified by the above screening methods in the preparation of a composition for use in the treatment of breast cancer and breast cancer metastasis including metastases in bone.

### Detailed Description of the Invention

In a first aspect, the invention provides diagnostic means to detect expression of cathepsin K in breast cells and tissues as well as in bone cells and tissues being indicative of developing or existing breast cancer and breast cancer metastasis to bony material, and other tissues respectively.

The means employed include nucleic acids encoding or relating to cathepsin K or fragments thereof, preferably cDNA or antisense RNA corresponding to the human cathepsin K gene and being capable of hybridising to the cathepsin K mRNA requiring at least 50% and preferably 70% sequence identity. The nucleic acid may be unlabelled or labelled with a detectable moiety.Thus, the nucleic acids according to the invention are useful e.g. in a method determining the presence of cathepsin K-specific nucleic acid, said method comprising hybridising the DNA (or RNA) encoding (or complementary to) cathepsin K to test sample nucleic acid and determining the presence of cathepsin K.

A method for amplifying a nucleic acid test sample comprising priming a nucleic acid polymerase (chain) reaction starting with RNA encoding cathepsin K is disclosed. Preferably, the PCR is performed using primers as depicted in SEQ ID Nos. 3 and 4.

The nucleic acids? whether used as probes or otherwise, are preferably substantially homologous to the sequence of cathepsin K as shown in SEQ ID No. 2. In a preferred embodiment, use is made of a DNA sequence as set forth in SEQ ID No. 1 or of a fragment thereof.

Preferably, nucleic acids are fragments of the cathepsin K-encoding sequence, or derivatives thereof. Fragments of the nucleic acid sequence of a few nucleotides in length, preferably 15 to 150 nucleotides in length, are especially useful as probes.

Exemplary nucleic acids can alternatively be characterised as those nucleotide sequences which encode a cathepsin K protein and hybridise to the DNA sequence set forth in SEQ ID No. 2, or a selected fragment of said DNA sequence. Preferred are such sequences encoding cathepsin K which hybridise under high-stringency conditions to the sequence of SEQ ID No. 2.

Stringency of hybridisation refers to conditions under which polynucleic acid hybrids are stable. Such conditions are evident to those of ordinary skill in the field. As known to those of skill in the art, the stability of hybrids is reflected in the melting temperature (Tₘ) of the hybrid which decreases approximately 1 to 1.5 °C with every 1 % decrease in sequence homology. In general, the stability of a hybrid is a function of sodium ion concentration and temperature. Typically, the hybridisation reaction is performed under conditions of higher stringency, followed by washes of varying stringency.

As used herein, high stringency refers to conditions that permit hybridisation of only those nucleic acid sequences that form stable hybrids in 1.M Na⁺ at 65-68 °C. High stringency conditions can be provided, for example, by hybridisation in an aqueous solution containing 6x SSC, 5x Denhardt's, 1 % SDS (sodium dodecyl sulphate), 0.1 Na⁺ pyrophosphate and 0.1 mg/ml denatured salmon sperm DNA as non specific competitor. Following hybridisation, high stringency washing may be done in several steps, with a final wash (about 30 minutes) at the hybridisation temperature in 0.2 - 0.1 x SSC, 0.1 % SDS.

Moderate stringency refers to conditions equivalent to hybridisation in the above described solution but at about 60-62 °C. In that case the final wash is performed at the hybridisation temperature in 1 x SSC, 0.1 % SDS.

Low stringency refers to conditions equivalent to hybridisation in the above described solution at about 50-52°C. In that case, the final wash is performed at the hybridisation temperature in 2x SSC, 0.1 % SDS.

It is understood that these conditions may be adapted and duplicated using a variety of buffers, e.g. formamide-based buffers, and temperatures. Denhardt's solution and SSC are well known to those of skill in the art as are other suitable hybridisation buffers (see, e.g. Sambrook, et al., eds. (1989) Molecular Cloning: A Laboratory Manual, Cold Spring Harbor Laboratory Press, New York or Ausubel, et al., eds. (1990) Current Protocols in Molecular Biology, John Wiley & Sons, Inc.). Optimal hybridisation conditions have to be determined empirically, as the length and the GC content of the probe also play a role.

Given the guidance provided herein, the nucleic acids are obtainable according to methods well known in the art. For example, a DNA of the invention is obtainable by chemical synthesis, using polymerase chain reaction (PCR) or by screening a genomic library or a suitable cDNA library prepared from a source believed to possess cathepsin K and to express it at a detectable level.

Chemical methods for synthesis of a nucleic acid of interest are known in the art and include triester, phosphite, phosphoramidite and H-phosphonate methods, PCR and other autoprimer methods as well as oligonucleotide synthesis on solid supports.

A nucleic acid encoding cathepsin K may be isolated e.g. by screening suitable cDNA or genomic libraries under suitable hybridisation conditions with a probe, i.e. a nucleic acid disclosed herein including oligonucleotides derivable from the sequence set forth in SEQ ID NO. 2. Suitable libraries are commercially available or can be prepared e.g. from cell lines, tissue samples, and the like.

As used herein, a probe is e.g. a single-stranded DNA or RNA that has a sequence of nucleotides that includes between 15 and 150, preferably between 15 and 30 and most preferably at least about 20 contiguous bases that are the same as (or the complement of) an equivalent or greater number of contiguous bases set forth in SEQ ID No. 2. The nucleic acid sequences selected as probes should be of sufficient length and sufficiently unambiguous so that false positive results are minimised. The nucleotide sequences are usually based on conserved or highly homologous nucleotide sequences or regions of cathepsin K. The nucleic acids used as probes may be degenerated at one or more positions.

Preferred regions from which to construct probes include 5' and/or 3' coding sequences, sequences predicted to encode ligand and/or substrate-specific binding sites, and the like. For example, either the full-length cDNA clone or fragments thereof represented in SEQ ID Nos. 1 and 2 or even fragments of the latter can be used as probes. Preferably, nucleic acid probes are labelled with suitable label means for ready detection upon hybridisation. For example, a suitable label means is a radiolabel. The preferred method of labelling a DNA fragment is by incorporating α^{32P} dATP with the Klenow fragment of DNA polymerase in a random priming reaction, as is well known in the art. Oligonucleotides are usually end-labelled with γ^{32P}-labelled ATP and polynucleotide kinase. However, other methods (e.g. non-radioactive) may also be used to label the fragment or oligonucleotide, including e.g. enzyme labelling, fluorescent labelling with suitable fluorophores and biotinylation.

After appropriate incubation of the sample to be examined for expression of cathepsin K with e.g. a portion of DNA including substantially the entire cathepsin K-encoding sequence or a suitable oligonucleotide based on a portion of said DNA as set forth in SEQ ID No.1, positive cells or tissues are identified by detecting a hybridisation signal. Also, based on the nucleic acid sequences provided herein antisense-type therapeutic agents may be designed.

In a most preferred embodiment, the nucleic acid which can be utilized as a probe is derived from a 455 bp *Eco* RI/*Sal* I cDNA fragment as set forth in SEQ ID No.1 corresponding to the 5' region of the human cathepsin K gene.

Furthermore, the means employed comprise antibodies specifically recognising and binding to cathepsin K or to fragments or other derivatives, or to analogs thereof displaying at least one antigenic determinant of human cathepsin K. For example, such antibodies may e.g. be generated against cathepsin K polypeptides having the amino acid sequence as encoded by the nucleotide sequence set forth in SEQ ID No. 2. Alternatively, cathepsin K or cathepsin K fragments (which may also be synthesised by *in vitro* methods known in the art) are fused (by recombinant expression or an *in vitro* peptidyl bond) to an immunogenic polypeptide and this fusion polypeptide, in turn, is used to raise antibodies against a human cathepsin K epitope.

Anti-cathepsin K antibodies may e.g. be recovered from the serum of immunised animals. Monoclonal antibodies may e.g. be prepared from cells from immunised animals in the conventional manner.

The antibodies are useful diagnostic means for the detection of the expression of cathepsin K in target cells or tissues to be examined.

Antibodies which can be utilized may e.g. be whole antibodies of natural classes, such as IgE, IgY and IgM antibodies, but are preferably IgG antibodies. Moreover, antibody fragments are disclosed, such as Fab, F(ab')₂, Fv and ScFv. Small fragments, such Fv and ScFv, possess advantageous properties for diagnostic and therapeutic applications on account of their small size and consequent superior tissue distribution.

The antibodies are especially indicated for diagnostic and therapeutic applications. Accordingly, they may be altered antibodies comprising an effector protein such as a toxin or a label. Especially preferred are labels which allow the imaging of the distribution of the antibody in a tumour *in vivo.* Such labels may e.g. be radioactive labels or radioopaque labels, such as metal particles, which are readily visualisable within the body of a patient. Moreover, they may be fluorescent labels or other labels which are visualisable on tissue samples removed from patients.

Recombinant DNA technology may be used to improve the antibodies. Thus, chimeric antibodies may be constructed in order to decrease the immunogenicity thereof in diagnostic or therapeutic applications. Moreover, immunogenicity may e.g. be minimised by humanising the antibodies by COR grafting [see European Patent Application 0 239 400 (Winter)] and, optionally, framework modification [see international patent application WO 90/07861 (Protein Design Labs)].

Antibodies may e.g. be obtained from animal serum, or, in the case of monoclonal antibodies or fragments thereof, produced in cell culture. Recombinant DNA technology may be used to produce the antibodies according to established procedure, in bacterial or preferably mammalian cell culture. The selected cell culture system preferably secretes the antibody product. For example, such antibodies may e.g. be obtained by culturing a host, e.g. E. coli or a mammalian cell, which has been transformed with a hybrid vector comprising an expression cassette comprising a promoter operably linked to a first DNA sequence encoding a signal peptide linked in the proper reading frame to a second DNA sequence encoding said protein, and isolating said protein.

Multiplication of hybridoma cells or mammalian host cells *in vitro* is carried out in suitable culture media, which are the customary standard culture media, for example Dulbecco's Modified Eagle Medium (DMEM) or RPMI 1640 medium, optionally replenished by a mammalian serum, e.g. fetal calf serum, or trace elements and growth sustaining supplements, e.g. feeder cells such as normal mouse peritoneal exudate cells, spleen cells, bone marrow macrophages, 2-aminoethanol, insulin, transferrin, low density lipoprotein, oleic acid, or the like. Multiplication of host cells which are bacterial cells or yeast cells is likewise carried out in suitable culture media known in the art, for example for bacteria in medium LB, NZCYM, NZYM, NZM, Terrific Broth, SOB, SOC, 2 x YT, or M9 Minimal Medium, and for yeast in medium YPD, YEPD, Minimal Medium, or Complete Minimal Dropout Medium.

*In vitro* production provides relatively pure antibody preparations and allows scale-up to give large amounts of the desired antibodies. Techniques for bacterial cell, yeast or mammalian cell cultivation are known in the art and include homogeneous suspension culture, e.g. in an airlift reactor or in a continuous stirrer reactor, or immobilised or entrapped cell culture, e.g. in hollow fibres, microcapsules, on agarose microbeads or ceramic cartridges.

Large quantities of the desired antibodies can also be obtained by multiplying mammalian cells *in vivo.* For this purpose, hybridoma cells producing the desired antibodies are injected into histocompatible mammals to cause growth of antibody-producing tumours. Optionally, the animals are primed with a hydrocarbon, especially mineral oils such as pristane (tetramethyl-pentadecane), prior to the injection. After one to three weeks, the antibodies are isolated from the body fluids of those mammals. For example, hybridoma cells obtained by fusion of suitable myeloma cells with antibody-producing spleen cells from Balb/c mice, or transfected cells derived from hybridoma cell line Sp2/0 that produce the desired antibodies are injected intraperitoneally into Balb/c mice optionally pre-treated with pristane, and, after one to two weeks, ascitic fluid is taken from the animals.

The cell culture supernatants are screened for the desired antibodies, preferentially by immunofluorescent staining of cells expressing cathepsin K, by immunoblotting, by an enzyme immunoassay, e.g. a sandwich assay or a dot-assay, or a radioimmunoassay.

For isolation of the antibodies, the immunoglobulins in the culture supernatants or in the ascitic fluid may be concentrated, e.g. by precipitation with ammonium sulphate, dialysis against hygroscopic material such as polyethylene glycol, filtration through selective membranes, or the like. If necessary and/or desired, the antibodies are purified by the customary chromatography methods, for example gel filtration, ion-exchange chromatography, chromatography over DEAE-cellulose and/or (immuno-)affinity chromatography, e.g. affinity chromatography with cathepsin K protein or with Protein-A.

The application discloses further hybridoma cells secreting the monoclonal antibodies of the invention. The preferred hybridoma cells are genetically stable, secrete monoclonal antibodies of the invention of the desired specificity and can be activated from deep-frozen cultures by thawing and recloning.

Furthermore, monoclonal antibodies directed against cathepsin K may e.g. be obtained by preparing a hybridoma cell line secreting said monoclonal antibodies, wherein a suitable mammal, for example a Balb/c mouse, is immunised with purified cathepsin K or an antigenic carrier containing purified cathepsin K. Antibody-producing cells of the immunised mammal can be fused with cells of a suitable myeloma cell line, and the hybrid cells obtained in the fusion can be cloned and cell clones secreting the desired antibodies can subsequently be selected.

Antibodies and antibody fragments are useful in diagnosis and therapy of breast cancer and breast cancer metastasis. Accordingly, a composition for therapy or diagnosis comprising an antibody is foreseen

In the case of a diagnostic composition, the antibody is preferably provided together with means for detecting the antibody, which may be enzymatic, fluorescent, radioisotopic or other means. The antibody and the detection means may be provided for simultaneous, simultaneous separate or sequential use, in a diagnostic kit intended for diagnosis.

As used herein, the terms "common structural" and "common antigenic" determinant, "fragment" and "derivative" of cathepsin K are to be understood to comprise the following meanings.

"Common structural determinant" means that the derivative in question displays at least one structural feature of cathepsin K. Structural features include possession of an epitope or antigenic site that is capable of cross-reacting with antibodies raised against a naturally occurring or denatured cathepsin K polypeptide or fragment thereof, possession of amino acid sequence identity with cathepsin K, and features having common a structure/function relationship. Thus, cathepsin K as referred to herein includes splice variants encoded by mRNA generated by alternative splicing of a primary transcript, amino acid mutants, glycosylation variants and other covalent derivatives of cathepsin K which retain the physiological and/or physical properties of cathepsin K. Exemplary derivatives include molecules wherein cathepsin K is covalently modified by substitution, chemical, enzymatic, or other appropriate means with a moiety other than a naturally occurring amino acid. Such a moiety may be a detectable moiety such as an enzyme or a radioisotope. Further included are naturally occurring variants of human cathepsin K. Such a variant may be encoded by a related gene of the same gene family, by an allelic variant of a particular gene, or represent an alternative splicing variant of the cathepsin K encoding gene.

Derivatives which retain common structural features can be fragments of cathepsin K. Fragments of cathepsin K comprise individual domains thereof, as well as smaller polypeptides derived from the domains. For example, smaller polypeptides derived from cathepsin K as encoded by the sequence represented in SEQ ID No. 2 define a single feature which is characteristic of cathepsin K. Fragments may in theory be almost any size, as long as they retain one feature of cathepsin K. Preferably, fragments will be between 5 and 200 amino acids in length. Longer fragments are regarded as truncations of the full-length cathepsin K and generally encompassed by the term "cathepsin K".

Derivatives of cathepsin K also comprise mutants thereof, which may contain amino acid deletions, additions or substitutions, subject to the requirement to maintain at least one feature characteristic of cathepsin K. Thus, conservative amino acid substitutions may be made substantially without altering the nature of cathepsin K, as may truncations from the 5' or 3' ends. Deletions and substitutions may moreover be made to the fragments of cathepsin K envisaged hereinbefore. Cathepsin K mutants may e.g. be produced from a DNA encoding cathepsin K which has been subjected to *in vitro* mutagenesis resulting e.g. in an addition, exchange and/or deletion of one or more amino acids. For example, substitutional, deletional or insertional variants of cathepsin K can be prepared by recombinant methods and screened for immuno-crossreactivity with the native forms of cathepsin K.

The fragments, mutants and other derivatives of cathepsin K preferably retain substantial homology with said protein. As used herein, "homology" means that the two entities share sufficient characteristics for the skilled person to determine that they are similar in origin and function. Preferably, homology is used to refer to sequence identity. Thus, the derivatives of cathepsin K preferably retain substantial sequence identity with the sequence encoded by the nucleotide sequence according to SEQ ID No. 2.

"Substantial homology", where homology indicates sequence identity, means more than 50% sequence identity, preferably more than 75% sequence identity and most preferably a sequence identity of 90% or more.

"Common antigenic determinant" means that the derivative in question displays at least one antigenic function of cathepsin K. Antigenic functions include possession of an epitope or antigenic site that is capable of cross-reacting with antibodies raised against a naturally occurring or denatured cathepsin K polypeptide or fragment thereof. Thus, cathepsin K as referred to herein includes splice variants encoded by mRNA generated by alternative splicing of a primary transcript, amino acid mutants, glycosylation variants and other covalent derivatives of cathepsin K which retain the physiological and/or physical properties of said protein. Exemplary derivatives include molecules wherein the protein is covalently modified by substitution, chemical, enzymatic, or other appropriate means with a moiety other than a naturally occurring amino acid. Such a moiety may be a detectable moiety such as an enzyme or a radioisotope. Further included are naturally occurring variants of human cathepsin K. Such a variant may e.g. be encoded by a related gene of the same gene family, by an allelic variant of a particular gene, or represent an alternative splicing variant of the cathepsin K gene.

Derivatives which retain common antigenic determinants can be fragments of cathepsin K. Such fragments comprise individual domains thereof, as well as smaller polypeptides derived from the domains. Preferably, smaller polypeptides derived from cathepsin K define a single epitope which is characteristic of said protein. Fragments may in theory be almost any size, as long as they retain one characteristic of cathepsin K. Preferably, fragments will be between 5 and 500 amino acids in length. Longer fragments are regarded as truncations of the full-length protein and generally encompassed by the term "cathepsin K".

Derivatives of cathepsin K also comprise mutants thereof, which may contain amino acid deletions, additions or substitutions, subject to the requirement to maintain at least one feature characteristic of said protein. Thus, conservative amino acid substitutions may be made substantially without altering the nature of cathepsin K, as may truncations from the 5' or 3' ends. Deletions and substitutions may moreover be made to the fragments of cathepsin K referred to herein. Cathepsin K mutants may e.g. be produced from a DNA encoding therefore which has been subjected to *in vitro* mutagenesis resulting e.g. in an addition, exchange and/or deletion of one or more amino acids. For example, substitutional, deletional or insertional variants of cathepsin K can be prepared by recombinant methods and screened for immuno-crossreactivity with the native forms of said protein.

The fragments, mutants and other derivative of cathepsin K preferably retain substantial homology with said protein. As used herein, "homology" means that the two entities share sufficient characteristics for the skilled person to determine that they are similar in origin and function. Preferably, homology is used to refer to sequence identity. Thus, the derivatives of cathepsin K preferably retain substantial sequence identity with the one encoded by the nucleotide sequence according to SEQ ID No. 2.

"Substantial homology", where homology indicates sequence identity, means more than 50% sequence identity, preferably more than 75% sequence identity and most preferably a sequence identity of 90% or more.

The novel identification of cathepsin K as a significant mediator of breast cancer and breast cancer metastasis permits the design of screening systems to identify putative therapeutic agents for use in treating and/or preventing breast cancer or breast cancer metastasis. Thus, in a second aspect of the invention, there is provided a method to screen for and evaluate potential antagonists for cathepsin K being capable of inhibiting or at least lowering cathepsin K activity with respect to breast cancer and breast cancer metastasis, comprising the steps of:
(a) incubating cathepsin K or a fragment thereof with the compound to be screened; and
(b) detecting interaction between the compound and cathepsin K or its fragment.

The screening may e.g. be carried out using complete cathepsin K or a fragment thereof, which preferably comprises at least part of the catalytic site and/or other domains responsible for binding its physiological targets and regulators.

Cathepsin K polypeptides or fragments may be isolated forms or complexed with further polypeptides. The compound to be screened may be present in essentially pure, uncomplexed form, or may e.g. be complexed with chemical groups for further polypeptides.

Incubation conditions will vary according to the precise method used to detect the interaction between the cathepsin K protein or fragment thereof and the screened compound. In the case of using transcription activation detection systems such as the yeast two-hybrid system, incubation conditions are suitable for gene transcription, such as those prevailing inside a living cell. Other detection systems, however, will require different incubation conditions. For example, if the detection of interaction is based on relative affinity in a chromatographic assay, for example as is known in affinity chromatographie, conditions will be adjusted to promote binding and then gradually altered, such that the point at which the screened compound no longer binds to the cathepsin K polypeptide or fragment thereof may be determined.

Incubation according to the invention may be achieved by a number of means, but the basic requirement is for cathepsin K or a fragment thereof and the screened compound to be able to come into contact with each other. This may e.g. be achieved by admixing cathepsin K or a fragment thereof and the compound, or by producing them *in situ,* such as by expression of nucleic acids encoding them. Where cathepsin K or a fragment thereof and/or the compound are in the form of fusions with other polypeptides, they may be expressed as such *in situ.*

Preferably, the method of the invention is based on a two-hybrid system. Such systems detect specific protein:protein interactions by exploiting transcriptional activators having separable DNA-binding and transcription activating domains, such as the yeast GAL4 activator. A reporter gene is operatively linked to an element responsive to the transcriptional activator being used, and exposed to cathepsin K or a fragment thereof and the compound to be screened, one of which is complexed to the transcription activating domain of the transcriptional activator and the other of which is joined to the DNA binding domain thereof. If there is a specific interaction between cathepsin K or a fragment thereof and the compound, the DNA binding and transcription activating domains of the transcriptional activator will be brought into juxtaposition and transcription from the reporter gene will be activated.

Alternatively, the detection may e.g. be based on observed binding between cathepsin K or a fragment thereof, such as its catalytic domain, and the screened compound, or a fragment thereof. In a similar manner, the involvement of cathepsin K on the activation or inactivation of a particular compound may be screened for by e.g. monitoring the interaction of a portion thereof known to be involved in modulation events with said protein.

Cathepsin K or a fragment thereof may e.g. be used to screen for compounds which bind thereto by incubating it with the compound to be screened and subsequently "pulling down" cathepsin K complexes with a cathepsin K-specific antibody. Antibodies suitable for immunoprecipitation or immuno-affinity chromatography have been described hereinbefore and may be prepared according to conventional techniques, known to those of ordinary skill in the art. For example, see Lane, et al., (1992) EMBO J., 11, 1743-1749. After the cathepsin K-compound complex has been isolated by affinity, the compound may be dissociated from the cathepsin K antibody and characterised by conventional techniques.

The interaction of cathepsin K or a fragment thereof with the screened compound may also be observed indirectly. For example, a modulator such as an inhibitor of cathepsin K function may be detected by observing the effects of cathepsin K on a substrate in the presence or absence of the compound. Preferred substrates for cathepsin K include collagen, osteonectin and a fluorogenic peptide substrate cbz - Phe - Arg - AMC. (Bossard, M.J. et al., loc. cit.)

The activity of cathepsin K or the catalytic domain thereof may e.g. be assessed by means of a protease activity assay, employing a substrate for the protease. Advantageously, proteolytic cleavage on cystein residues normally implicated in protease function is assessed.

The assay of the invention may e.g. be used to measure the direct effect of the candidate compound on cathepsin K.

In order to obtain a meaningful result, the activity of cathepsin K exposed to the candidate agent should be compared to the activity of cathepsin K not exposed to the agent, an inhibitory or eliminating effect on cathepsin K protease activity being indicative of potential as an agent suitable for blocking or at least lowering cathepsin K activity in breast cancer cells thus reducing the possibility of metastasis.

Promising compounds may then be further assessed by determining the properties thereof directly, for instance by means of a cellular screening assay using breast cancer cell lines such as MCF7 (ATTC accession no. HTB-22), which preferably express relatively high levels of cathepsin K activity. Such an assay preferably involves comparing the inhibitory potential of the compound to be screened with that of an already known inhibitory compound. For this purpose, E64 and leupeptin, which have been reported as inhibitors of cystein proteases including cathepsin K (Bossard, M.J. et al., Proteolytic activity of human osteoclast cathepsin K, J. Biol. Chem., 271:12517-12524, 1996) can be used as model substances.

In a similar manner, the qualification and inhibitory potential of other molecules such as antisense oligonucleotides directed at specific sites within the cathepsin K gene or neutralising antibodies which can be used according to the invention can be assessed under the same conditions.

Kits useful for screening such compounds may be prepared, and will comprise essentially cathepsin K or a fragment thereof together with means for detecting an interaction between said protease and the screened compound. Preferably, therefore, the screening kit comprises one of the detection systems set forth hereinbefore.

Cathepsin K for use in kits may be provided in the form of a protein, for example in solution, suspension or lyophilised, or in the form of a nucleic acid sequence permitting the production of cathepsin K or a fragment thereof in an expression system, optionally *in situ.* Preferably, the nucleic acid encoding cathepsin K or a fragment thereof encodes it in the form of a fusion protein, for example a GST fusion.

Compounds may be identified by screening using the techniques described hereinbefore, and prepared by extraction from natural sources according to established procedures, or by synthesis, especially in the case of low molecular weight chemical compounds. Proteinaceous compounds may e.g. be prepared by expression in recombinant expression systems, for example a baculovirus system as available e.g. from Invitrogen and Clontech, or in a bacterial system, for example as described in UK patent application 9525705.1. Proteinaceous compounds are mainly useful for research, although they may have a therapeutic application.

Low molecular weight compounds, on the other hand, are preferably produced by chemical synthesis according to established procedures. They are primarily indicated as therapeutic agents. Low molecular weight compounds and organic compounds in general may be useful as cathepsin K mimics or antimetastasizing agents.

Those compounds which display a desired inhibitory effect on cathepsin K activity in one or more of the assays mentioned above can then be subjected to an *in vivo* screening using either a rat or mouse model. Stable transfectants of the full length cathepsin K, ligated into a suitable mammalian expression vector and transfected into a cathepsin K negative murine tumorigenic cell line are selected and used for injection of mice or rats in the presence or absence of inhibitors for investigation and evaluation of metastasis inhibition.

Accordingly, the present invention also provides use of the antisense nucleic acids and antibodies disclosed hereinbefore in the preparation of a composition for use in the treatment of breast cancer and breast cancer metastasis.

The pharmaceutical preparations are those for enteral, such as oral, furthermore rectal, and parenteral administration to women, the pharmacological active ingredient being present on its own or together with a pharmaceutically acceptable carrier. The daily dose of the active ingredient depends on the age and the individual condition and also on the manner of administration.

The pharmaceutical preparations contain, for example, from about 10 % to about 80%, preferably from about 20 % to about 60 %, of the active ingredient. Pharmaceutical preparations for enteral or parenteral administration are, for example, those in unit dose forms, such as sugar-coated tablets, tablets, capsules or suppositories, and furthermore ampoules. These are prepared in a manner known per se, for example by means of conventional mixing, granulating, sugar-coating, dissolving or lyophilizing processes. Thus, pharmaceutical preparations for oral use can be obtained by combining the active ingredient with solid carriers, if desired granulating a mixture obtained, and processing the mixture or granules, if desired or necessary, after addition of suitable excipients to give tablets or sugar-coated tablet cores.

Suitable carriers are, in particular, fillers, such as sugars, for example lactose, sucrose, mannitol or sorbitol, cellulose preparations and/or calcium phosphates, for example tricalcium phosphate or calcium hydrogen phosphate, furthermore binders, such as starch paste, using, for example, corn, wheat, rice or potato starch, gelatin, tragacanth, methylcellulose and/or polyvinylpyrrolidone, if desired, disintegrants, such as the abovementioned starches, furthermore carboxymethyl starch, crosslinked polyvinylpyrrolidone, agar, alginic acid or a salt thereof, such as sodium alginate; auxiliaries are primarily glidants, flow-regulators and lubricants, for example silicic acid, talc, stearic acid or salts thereof, such as magnesium or calcium stearate, and/or polyethylene glycol. Sugar-coated tablet cores are provided with suitable coatings which, if desired, are resistant to gastric juice, using, inter alia, concentrated sugar solutions which, if desired, contain gum arabic, talc, polyvinylpyrrolidone, polyethylene glycol and/or titanium dioxide, coating solutions in suitable organic solvents or solvent mixtures or, for the preparation of gastric juice-resistant coatings, solutions of suitable cellulose preparations, such as acetylcellulose phthalate or hydroxypropylmethylcellulose phthalate. Colorants or pigments, for example to identify or to indicate different doses of active ingredient, may be added to the tablets or sugar-coated tablet coatings.

Other orally utilizable pharmaceutical preparations are hard gelatin capsules, and also soft closed capsules made of gelatin and a plasticizer, such as glycerol or sorbitol. The hard gelatin capsules may contain the active ingredient in the form of granules, for example in a mixture with fillers, such as lactose, binders, such as starches, and/or lubricants, such as talc or magnesium stearate, and, if desired, stabilizers. In soft capsules, the active ingredient is preferably dissolved or suspended in suitable liquids, such as fatty oils, paraffin oil or liquid polyethylene glycols, it also being possible to add stabilizers.

Suitable rectally utilizable pharmaceutical preparations are, for example, suppositories, which consist of a combination of the active ingredient with a suppository base. Suitable suppository bases are, for example, natural or synthetic triglycerides, paraffin hydrocarbons, polyethylene glycols or higher alkanols. Furthermore, gelatin rectal capsules which contain a combination of the active ingredient with a base substance may also be used. Suitable base substances are, for example, liquid triglycerides, polyethylene glycols or paraffin hydrocarbons.

Suitable preparations for parenteral administration are primarily aqueous solutions of an active ingredient in water-soluble form, for example a water-soluble salt, and furthermore suspensions of the active ingredient, such as appropriate oily injection suspensions, using suitable lipophilic solvents or vehicles, such as fatty oils, for example sesame oil, or synthetic fatty acid esters, for example ethyl oleate or triglycerides, or aqueous injection suspensions which contain viscosity-increasing substances, for example sodium carboxymethylcellulose, sorbitol and/or dextran, and, if necessary, also stabilizers.

The dose of the active ingredient depends on the age and the individual condition of the individual to be administered and on the manner of administration. In the normal case, an approximate daily dose of about 10 mg to about 250 mg is to be estimated in the case of oral administration for a patient weighing approximately 75 kg .

The invention is further described, for the purposes of illustration only, in the following examples.

### Examples

### Preparation of frozen tissue sections

Samples are obtained at surgery from patients with breast carcinoma or breast carcinoma with skeletal metastases.

Fresh tissue from these surgical procedures is removed and cut into approximately 0.5cm³ pieces with surgical instruments. These are then submerged in a beaker of prechilled n-hexane surrounded by dry ice for 1 minute.

Using sterile forceps, the tissue blocks are transferred into sterile 50 ml falcon tubes and stored at -70 °C until use. A cryostat is cooled to -35 °C and the RNase free knife is chilled to -18 °C. The tissue is carefully placed onto a drop of tissuetek solution (Miles Inc., Diagnostics Div., Elkhart, IN, USA) on a mounting block. The block is placed in dry ice so that the mounting fluid solidifies and the tissue is held in place. The block is placed in the cryostat and sections of 10 µm are cut and mounted onto TESPA (SIGMA) coated slides.

### 3-aminopropyltriethoxysilane TESPA coating of slides

The slides are handled with gloves and prepared by dipping into the following solutions for 10 seconds each:
- 10% (v/v) HCI in 70% (v/v) alcohol
- distilled water
- 95% (v/v) alcohol.

The slides are then dried in an oven at 150 °C and allowed to cool before dipping into the following solutions for 10 seconds each:
- 2% (v/v) TESPA in acetone
- 100% acetone for 2 times
- distilled water.

The slides are baked dry in an oven at 42 °C, transferred to a dessicated box and stored at 4 °C. The sections are allowed to air dry for 5 minutes, then treated with 4% paraformaldehyde (w/v) in DEPC treated PBS for 5 minutes at room temperature, washed several times in DEPC/PBS and air dried. The slides are individually wrapped and stored at -70 °C until use.

### Radioactive detection by in situ hybridization

### Preparation of RNA probes

Approximately 6 µg of plasmid construct (using Bluescript SK⁺, Stratagene) is linearized with two separate enzymes in two separate tubes.

| | | |
|---|---|---|
| e.g. | Cathepsin K 6 µg | Cathepsin K 6 µg |
| | Buffer H 3 µl | Buffer H (10 X stock) 3 µl |
| | *Sal* I 2 µl | *Eco* RI 2 µl |
| | Volume made to 30 µl | Volume made to 30 µl |

After 3 hours the reaction volume is increased to 100 µl with water and 50 µl of phenol, 50 µl chloroform is added prior to vortexing and centrifuging for 15 minutes at 14,000 rpm. The top layer is transferred to an RNAse free tube. The linearized DNA is precipitated with one tenth volume of 3 M sodium acetate and 2 volumes of absolute ethanol. After brief vortexing, the preparation is left overnight to precipitate at -20 °C.

On the following day, the DNA is centrifuged 15 minutes at 14,000 rpm and washed 2 times with 70% ethanol, and once with absolute ethanol. The sediment is air dried at 50 °C for 2 minutes and resuspended in 20 µl of DEPC water. An aliquot of 2 µl is run on a 1 % agarose gel with ethidium bromide and observed under UV to check yield and quality of the preparation.

The equivalent of 0.5 µg is transferred to an RNAse free tube and the SP6 T7 labelling kit from Boehringer is used. The following reagents are added in this order:
- 0.5 µg linearized DNA
- 3 µl of NTP labelling mixture (ATP, UTP, GTP)
- 2 µl buffer
- 1 µl RNase inhibitor
- 1 µl of ³⁵S CTP
- DEPC treated water to 18 µl
- 2 µl of either T3, T7 or SP6 RNA polymerase.

This is incubated for 2 hours at 37 °C and after this time, 2 µl of DNAse (RNase free) is added for further 15 minutes at 37 °C. 2 µl of 0.2 M EDTA is added followed by precipitation with 20 µl of 5 M ammonium acetate, 1 µl glycogen and 250 µl of prechilled ethanol. After thorough mixing, the preaparation is incubated overnight at -20 °C. The RNA is washed with 70°% ethanol and air dried at 50 °C for 2 minutes. This is then resuspended in 20 µl of DEPC water and 0.5 µl spotted onto filter paper, placed in a scintillation vial with 5 ml of scintillant, and counted.

### Prehybridization

The sections are rehydrated twice in DEPC/PBS for 3 minutes each, and DEPC/PBS/0.1 M glycine for 5 minutes at room temperatur, followed by another incubation in DEPC/PBS for 3 minutes. The sections are then treated with citric acid buffer for 1 hour at room temperatur followed by proteinase K (1 µg/ml in 0.1 M Tris 50 mM EDTA, pH 8) for 20 minutes at 37 °C to allow access of the RNA probes and decrease non-specific binding. This is followed by a paraformaldehyde treatment (4% paraformaldehyde in DEPC treated PBS) for 5 minutes to retain cellular localization of the mRNA and application of acetic anhydride (0.25% v/v in 0.1 M DEPC/triethanolamine) to acetylate any remaining proteins. The sections are incubated in 50 % formamide, 2 X SSC for 10 minutes before the solution is carefully removed from the slide surrounding the sections. 10-20 µl of prehybridization solution is subsequently added to the sections, 50% formamide, 2 X SSC, 5 mg/ml tRNA, 200 µg/ml ssDNA, 10 % dextran sulphate, 1 X Denhardts solution and 10 mM DTT.

### Hybridization

Prehybridization is carried out in a humidified box at 42 °C for 3 hours before addition of 300,000 cpm of sense or antisense RNA probe per section. The sections are then carefully overlayed with parafilm squares taking care that sense and antisense sections do not intermingle. Hybridization is performed overnight at 42 °C.

### Post hybridization washes

The sections are removed from the humidified box and transferred to a coplin jar containing 4 X SSC for 30 minutes at room temperatur. The parafilm squares are gently removed at this point. The sections are carefully washed at 37 °C in a shaking water bath with the following solutions:
- 4 X SSC for 4 times, 15 minutes each
- 0.5 M NaCI/10 mM Tris/1 mM EDTA for 10 minutes
- 0.5 M NaCI/10 mM Tris/1 mM EDTA and 20 µg/ml RNAse A for 30 minutes
- 2 X SSC for 30 minutes
- 0.1 X SSC for 30 minutes.

After washing the sections are dehydrated in 70%, 95% and finally 100% ethanol each containing 0.3 M ammonium acetate for 5 minutes per solution and left to air dry.

### Exposure

In a dark room LM1 emulsion (Amersham) is warmed at 43° C in a waterbath before pouring into a dipping chamber (Amersham). The slides are dipped in LM1 emulsion and then placed on a sheet of ice cold metal for 10 minutes to allow the emulsion to start solidifying. The slides are removed and stood upright and allowed to dry before storage at 4 °C for 10 days in a light proof box containing silica dessicant packets to ensure that the slides remain dry. Development is performed in Kodak D19 (Sigma) developer for 3 minutes followed by a treatment in water for 10 seconds and fixation in 30 % (w/v) sodium thiosulphate for 10 minutes. The tissues are counterstained in methylene blue, mounted and photographed.

### Non radioactive detection by in situ hybridization

### Preparation of RNA probes

Approximately 6 µg of plasmid construct is linearized with two separate enzymes in two separate tubes.

| | | |
|---|---|---|
| e.g. | Cathepsin K 6 µg | Cathepsin K 6 µg |
| | Buffer H 3 µl | Buffer H (10 X stock) 3 µl |
| | *Sal* l 2 µl | *Eco* RI 2 µl |
| | Volume made to 30 µl | Volume made to 30 µl. |

After 3 hours the reaction volume is increased to 100 µl with water and 50 µl of phenol, 50 µl chloroform is added prior to vortexing and centrifuging for 15 minutes at 14,000 rpm. The top layer is transferred to an RNAse free tube. The linearized DNA is precipitated with one tenth volume 3 M sodium acetate and 2 volumes of absolute ethanol. Subsequently, the preparation is vortexed and left overnight to precipitate at -20 °C.

On the following day, the DNA is centrifuged for 15 minutes at 14,000 rpm and washed 2 times with 70% ethanol, and once with absolute ethanol. The sediment is air dried at 50 °C for 2 minutes and resuspended in 20 µl of DEPC water. An aliqout of 2 µl is run on a 1% agarose gel with ethidium bromide and observed under UV to check yield and quality of the preparation.

The equivalent of 1 µg is transferred to a RNAse free tube and the DIG labelling kit from Boehringer is used. The following reagents are added in this order:
- 1 µg linearized DNA
- 2 µl of NTP labelling mixture (ATP, CTP, GTP DIGUTP)
- 2 µl buffer
- 1 µl RNase inhibitor
- 2 µl of either T3, T7 or SP6 RNA polymerase.

This is incubated for 2 hours at 37 °C and after this time, 2 µl of DNAse (RNase free) is added for further 15 minutes at 37 °C. 2 µl of 0.2 M EDTA is added followed by precipitation with 2,5 µl of 4 M LiCl and 75 µl prechilled ethanol. After thorough mixing, the preparation is incubated overnight at -20 °C, or for several hours at least. The RNA is washed with 70% ethanol and air dried at 50 °C for 2 minutes. This is then resuspended in 50 µl of DEPC water and quantified. The concentration is 100 ng per µl.

### Quantification of DIG-RNA probes

An aliqout of 1 µl is diluted to 5 µl with water to result in a yield of 20 ng/µl. Control labelled RNA in the DIG labelling kit is diluted to 20 ng/µl.

The following dilutions are made in 50% DEPC water, 30% 20 X SSC, 20% formaldehyde.

| | | |
|---|---|---|
| 20 ng/µl | 2 µl/38 µl solution | 1 ng/µl A |
| 1 ng/µl | 5 µl/45 µl solution | 100 pg/µl B |
| 100 pg/µl | 5 µl/45 µl solution | 10 pg/µl C |
| 10 pg/µl | 5 µl/45 µl solution | 1 pg/µl D |
| 1 pg/µl | 5 µl/45 µl solution | 0.1 pg/µl E |
| 0.1 pg/µl | 5 µl/45 µl solution | 0.01 pg/µl F |

1 µl of dilutions B-F are spotted on to DIG quantification test strips Cat. No. 1669 966 (Boehringer) and allowed to air dry for 2 minutes.

In the meantime, the following solutions are prepared:
(DIG detection kit Cat. No. 1175 041) (Boehringer)
Buffer 1: 0.1 M maleic acid, 0.15 M NaCl, pH 7.5.
Blocking stock solution: blocking reagent in the kit is dissolved in 0.1 M maleic acid, 0.15 M NaCl, pH 7.5.
Blocking solution: Blocking stock solution is dissolved 1:10 in 0.1 M maleic acid, 0.15 M NaCl, pH 7.5.
Washing buffer: 0.1 M maleic acid, 0.15 M NaCl, pH 7.5 and 0.3% w/v Tween 20.
Buffer 3: 100 mM TrisCl, 100 mM NaCl, 50 mM MgCl₂, pH 9.5.

A set of 15 ml falcon tubes containing approximately 3 ml of the following solutions is then prepared:
- tube 1: blocking solution
- tube 2: antibody binding (1.5 µl of the anti DIG antibody in the kit in 3 ml blocking solution)
- tube 3: blocking solution
- tube 4: washing buffer
- tube 5: buffer 3
- tube 6: colour reaction (66 µl of the NBT/BClP solution in the kit with 3 ml of buffer 3).

The strips are dipped sequentially into:
tube 1 for 2 minutes
tube 2 for 3 minutes
tube 3 for 1 minute
tube 4 for 1 minute
tube 5 for 1 minute
tube 6 for 5-30 minutes.

The spots are then compared to the known control strip and the yield of DIG labelled RNA is worked out.

### Prehybridization

Paraffin sections are baked on a hot plate at 50 °C for 10 minutes and then resuspended (in DEPC water pre-treated coplin jars) twice in xylol for 3 minutes followed by absolute ethanol for 2 X 2 minutes, 95% ethanol for 2 minutes, 85% ethanol for 2 minutes, 70% ethanol for 2 minutes, 50% ethanol for 2 minutes and DEPC treated PBS for 2 minutes. The sections are refixed in 4% paraformaldehyde in PBS/DEPC for 5 minutes and washed once in DEPC PBS for 3 minutes. The frozen sections can be added at this stage and are incubated in the following solutions:
- DEPC PBS, 2 x 5 minutes
- DEPC PBS/0.1 M glycine, 2 x 5 minutes
- DEPC PBS 0.3% Triton X-100, 15 minutes
- DEPC PBS, 2 x 5 minutes
- 100 mM Tris, 50 mM EDTA, pH 8, 1 µg/ml Proteinase K, 30 minutes at 37 °C
- DEPC PBS with 4% paraformaldehyde, 5 minutes
- DEPC PBS, 2 x 5 minutes
- 0.1 M triethanolamine with freshly added acetic anhydride 0.25% v/v, 2 x 5 minutes
- 2 X SSC, 50% formamide, 10 minutes.

The sections are then wiped around carefully and prehybridisation solution is added to cover them:
- 50% formamide
- 2XSSC
- 200 µg/ml ssDNA
- 10 mM DTT
- 10% dextran sulfate
- 1 µl RNAse inhibitor/ml
- 5 mg/ml tRNA
- 1 X Denhardts solution.

### Hybridization

Prehybridization is carried out in a humidified box at 42 °C for at least 1 hour before addition of 5 to 10 ng of digoxygenin labelled sense or antisense RNA probe per section. The sections are then carefully overlayed with a square of parafilm taking care that sense and antisense sections do not intermingle. Hybridization is performed overnight at 42 °C.

### Posthybridization washes

The coverslips are removed by immersion of the slides into 2 X SSC for 5 - 10 minutes. The slides are carefully washed at 37 °C in a shaking water bath with the following solutions:
- 2 X SSC for 2 times, 15 minutes each
- 1 X SSC for 2 times, 15 minutes each
- 0.5 M NaCl/10 mM Tris/1 mM EDTA, pH 8, containing 20 µg/ml RNAse A, 30 minutes
- 0.1 X SSC for 2 times, 30 minutes each.

### Immunological detection

The sections are washed in:
- Buffer 3 for 2 x 10 minutes on a shaking platform at room temperatur
- blocking solution with 1 % normal sheep serum and 0.1 % Triton X 100 for 30 minutes
- the previous solution containing 1:5000 dilution of anti DIG antibody for 2 hours in a humidified box at room temperatur
- Buffer 3 for 2 x 10 minutes
- Buffer 3 containing the colour reaction (200 µl NBT/BCIP per 10 ml of buffer 3) in a humidified box in the dark for 2 - 24 hours.

The reaction is stopped in buffer 4 (10 mM Tris, 1 mM EDTA, pH 8.1). Then, the slides are dipped briefly in water, counterstained, mounted and photographed.

### Detection by immunostaining

An anti-cathepsin K antibody from IRL, Japan is used (Littlewood-Evans A. et al., Localization of Cathepsin K in Human Osteoclasts by in situ Hybridization and Immunohistochemistry, Bone, Vol. 20, 1997).

Frozen and paraffin embedded sections are used for this procedure. Blocks of tissue are fixed with 4% paraformaldehyde in PBS for 24 hours prior to paraffin embedding. Paraffin sections (5 µm) are mounted onto Vectabond (Vector Labs, UK) treated slides and dried overnight in a 50 °C oven before dewaxing with xylene. 10 µm frozen sections on TESPA slides are post fixed with acetone for 30 seconds.

At a concentration of 1 mg/ml, the anti-cathepsin K antibody revealed optimal binding to purified cathepsin K by ELISA (data not shown), therefore this concentration was used for immunohistochemical localization studies.

Endogenous peroxidase activity is inhibited by immersing the sections in 1 % H₂O₂ in PBS for 30 minutes. After rinsing in PBS, the sections are flooded with swine serum for 30 minutes to block non-specific staining. Chicken anti-cathepsin K antibody is diluted in PBS to 1 mg/ml and applied to the sections in a humidified box for 60 minutes. Following thorough washing in PBS, a 1:500 dilution of a second antibody, rabbit anti-chicken IgG whole molecule peroxidase conjugate (Sigma) is incubated with the sections for 60 minutes. The tissues are washed as before and treated with Sigma fast DAB tablets according to the manufacturer's instructions for approximately 1 minute prior to rinsing in distilled water and counterstaining with Meyers haematoxylin.

### RT-PCR of breast cancer cell lines

Breast cancer cell lines purchased from ATCC unless otherwise stated, are classified into subtypes: infiltrating ductal carcinoma - ZR75-1 (CRL-1500), Hs578T duct (HTB-126), BT474 (HTB-20), ZR75-30 (CRL-1504), BT 549 (HTB-122) and T-47D (HTB-133); adenocarcinoma - MDA-MB-231 (HTB-132), SK-BR3 (HTB-30), MDA-MB-468 (HTB-132) and BT20 (HTB-19); and breast carcinoma - MVLN, MTLN (these two lines were obtained from Dr JC Nicolas, Unit de Recherches sur la Biochemie des Steroides, Insern U58 Montpelier, France) and MCF-7 (HTB-22).

Other breast lines consisted of HBL-100 (HTB-124) breast epithelial line isolated from the milk of a nursing mother. At higher passage numbers, the cells could become tumorigenic in nude mice and therefore can not be considered normal. MCF10A (CRL-10317), derived from mammary tissue of a patient with fibrocystic breast disease, expresses a normal or near normal karyotype. HMEC4144 (Clonetics, 9620 Chesapeake Drive Suite 201, San Diego, CA 92123, USA) is a human mammary epithelial cell line considered normal. MTSV1.7neo is a non malignant human breast derived cell line (Bartek J., et al., Efficient immortalization of luminal epithelial cells from human mammary gland by introduction of simian virus 40 large tumour antigen with a recombinant retrovirus, Pro. Natl. Acad Sci., 88: 3520 - 3524, 1991).

### PCR analysis

Total RNA is extracted from 14 breast cancer cell lines using a TRIzol kit (GIBCO BRL) which isolates pure RNA ensuring no co-purification of DNA. Approximately 35 µg of total RNA is then reverse transcribed according to the manufacturers instructions (Promega) in a total volume of 200 µl The RT reaction mixture consists of 40 µl MgCl₂ (25 mM final concentration), 20 µl 10 X transcription buffer, 20 µl dNTPs mixture (1 mM each), 5 µl RNasin (1 U/µl), 5 µl AMV reverse transcriptase (30 U/µl), 10 µl of oligo dT mixture (5 µg) and is made up to a final volume of 200 µl with nuclease free water. The RT mixture is incubated at 42 °C for 15 minutes and terminated by placing the mixture on ice. The cDNA is aliquoted and stored at -20 °C until required. An aliqout of 3 µl is subsequently used for PCR analysis. Two primers for cathepsin K spanning large introns in the genomic sequence thus eliminating the possibility of genomic DNA contamination in the original RNA preparation are designed and used for the amplification reactions:
Forward primer 5' TTC CCG CAG TAA TGA CAC C SEQ ID No. 3
Reverse primer 5' TTT CCC CAG TTT TCT CCC C SEQ ID No. 4.

The expected product size is 615 bp. In a 100 µl reaction volume, the following components are added: 10 µl PCR buffer (10 X stock, Boehringer Mannheim), 5 µl DMSO, 2 µl dNTP mix (10 mM stock), 0.5 µg of each primer, 0.5 µl Taq polymerase (Boehringer Mannheim), water to 97 µl and 3 µl of reverse transcribed RNA.

The PCR cycling conditions used are 94 °C for 3 minutes for 1 cycle, followed by 35 cycles of 94 °C for 1 minute, 48 °C for 1.5 minutes and 72 °C for 2.5 minutes. At the end of the cycling a further extension period of 10 minutes at 72 °C is performed and the PCR reactions stored at 4 °C.

The PCR reactions are electrophoresed on a 1 % agarose gel and the bands are visualized with ethidium bromide.

### Southern blot analysis

The DNA on the agarose gel is transferred overnight onto a Hybond membrane (Amersham) in 0.4 M NaOH and 0.6 M NaCl. The membrane is hybridized at 42 °C overnight with a full length cathepsin K cDNA probe labelled with ³²P. On the following day, the membrane is washed with 2 X SSC/0.5% SDS for 30 minutes at 42 °C followed by another wash in fresh washing solution at 50 °C for 30 minutes. The membrane is wrapped and exposed for autoradiography for 3-6 hours.

### SEQUENCE LISTING

(1) GENERAL INFORMATION:
   (i) APPLICANT:
      (A) NAME: NOVARTIS AG
      (B) STREET: SCHWARZWALDALLEE
      (C) CITY: Basel
      (E) COUNTRY: Switzerland
      (F) POSTAL CODE (ZIP): 4002
      (G) TELEPHONE: +41 61 696 11 11
      (H) TELEFAX: + 41 61 696 79 76
      (I) TELEX: 962 991
   (ii) TITLE OF INVENTION: NOVEL METHODS
   (iii) NUMBER OF SEQUENCES: 4
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
(2) INFORMATION FOR SEQ ID NO: 1:
   (i) SEQUENCE CHARACTERISTICS:
      (A) LENGTH: 455 base pairs
      (B) TYPE: nucleic acid
      (C) STRANDEDNESS: double
      (D) TOPOLOGY: linear
   (ii) MOLECULE TYPE: cDNA
   (xi) SEQUENCE DESCRIPTION: SEQ ID NO: 1:
   ATGTGGGGGC TCAAGGTTCT GCTGCTACCT GTGGTGAGCT TTGCTCTGTA CCCTGAGGAG 60
   ATACTGGACA CCCACTGGGA GCTATGGAAG AAGACCCACA GGAAGCAATA TAACAACAAG 120
   GTGGATGAAA TCTCTCGGCG TTTAATTTGG GAAAAAAACC TGAAGTATAT TTCCATCCAT 180
   AACCTTGAGG CTTCTCTTGG TGTCCATACA TATGAACTGG CTATGAACCA CCTGGGGGAC 240
   ATGACCAGTG AAGAGGTGGT TCAGAAGATG ACTGGACTCA AAGTACCCCT GTCTCATTCC 300
   CGCAGTAATG ACACCCTTTA TATCCCAGAA TGGGAAGGTA GAGCCCCAGA CTCTGTCGAC 360
   TATCGAAAGA AAGGATATGT TACTCCTGTC AAAAATCAGG GTCAGTGTGG TTCCTGTTGG 420
   GCTTTTAGCT CTGTGGGTGC CCTGGAGGGC CAACT 455
(2) INFORMATION FOR SEQ ID NO: 2:
   (i) SEQUENCE CHARACTERISTICS:
      (A) LENGTH: 990 base pairs
      (B) TYPE: nucleic acid
      (C) STRANDEDNESS: double
      (D) TOPOLOGY: linear
   (ii) MOLECULE TYPE: cDNA
   (xi) SEQUENCE DESCRIPTION: SEQ ID NO: 2:
   ATGTGGGGGC TCAAGGTTCT GCTGCTACCT GTGGTGAGCT TTGCTCTGTA CCCTGAGGAG 60
   ATACTGGACA CCCACTGGGA GCTATGGAAG AAGACCCACA GGAAGCAATA TAACAACAAG 120
   GTGGATGAAA TCTCTCGGCG TTTAATTTGG GAAAAAAACC TGAAGTATAT TTCCATCCAT 180
   AACCTTGAGG CTTCTCTTGG TGTCCATACA TATGAACTGG CTATGAACCA CCTGGGGGAC 240
   ATGACCAGTG AAGAGGTGGT TCAGAAGATG ACTGGACTCA AAGTACCCCT GTCTCATTCC 300
   CGCAGTAATG ACACCCTTTA TATCCCAGAA TGGGAAGGTA GAGCCCCAGA CTCTGTCGAC 360
   TATCGAAAGA AAGGATATGT TACTCCTGTC AAAAATCAGG GTCAGTGTGG TTCCTGTTGG 420
   GCTTTTAGCT CTGTGGGTGC CCTGGAGGGC CAACTCAAGA AGAAAACTGG CAAACTCTTA 480
   AATCTGAGTC CCCAGAACCT AGTGGATTGT GTGTCTGAGA ATGATGGCTG TGGAGGGGGC 540
   TACATGACCA ATGCCTTCCA ATATGTGCAG AAGAACCGGG GTATTGACTC TGAAGATGCC 600
   TACCCATATG TGGGACAGGA AGAGAGTTGT ATGTACAACC CAACAGGCAA GGCAGCTAAA 660
   TGCAGAGGGT ACAGAGAGAT CCCCGAGGGG AATGAGAAAG CCCTGAAGAG GGCAGTGGCC 720
   CGAGTGGGAC CTGTCTCTGT GGCCATTGAT GCAAGCCTGA CCTCCTTCCA GTTTTACAGC 780
   AAAGGTGTGT ATTATGATGA AAGCTGCAAT AGCGATAATC TGAACCATGC GGTTTTGGCA 840
   GTGGGATATG GAATCCAGAA GGGAAACAAG CACTGGATAA TTAAAAACAG CTGGGGAGAA 900
   AACTGGGGAA ACAAAGGATA TATCCTCATG GCTCGAAATA AGAACAACGC CTGTGGCATT 960
   GCCAACCTGG CCAGCTTCCC CAAGATGTGA 990
(2) INFORMATION FOR SEQ ID NO: 3:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "PRIMER"
   (xi) SEQUENCE DESCRIPTION: SEQ ID NO: 3:
      TTCCCGCAGT AATGACACC 19
(2) INFORMATION FOR SEQ ID NO: 4:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "PRIMER"
   (xi) SEQUENCE DESCRIPTION: SEQ ID NO: 4:
      TTTCCCCAGT TITCTCCCC 19

## Claims

1. A method to screen for antagonists of the polypeptide encoded by SEQ. ID. No. 2 capable of inhibiting or at least lowering the activity of the polypeptide for treating breast cancer and breast cancer metastasis, comprising the steps of:
(a) incubating a polypeptide encoded by from SEQ ID No. 2 or a fragment thereof with the compound to be screened; and
(b) detecting interaction between the compound and the polypeptide or its fragment.

2. Method according to claim 1, wherein the polypeptide Segment is selected from the catalytic domain.

3. Use of an antibody or immunologically active fragment thereof specific for a polypeptide encoded by SEQ ID No. 2 for the manufacture of a diagnostic for breast cancer and breast cancer metastasis.

4. Use according to claim 3, wherein the polypeptide fragment is selected from the catalytic domain.

5. A method of diagnosing breast cancer or breast cancer metastasis in which the expression of the polypeptide encoded by SEQ. ID. No. 2 is determined with a nucleic acid having SEQ. ID. No. 2 or a fragment thereof.

6. A method as claimed in claim 5, wherein the nucleic acid is DNA or RNA.

7. A method as claimed in claim 5 or 6, wherein the nucleic acid has a sequence as indicated in SEQ ID No. I.

8. Use of an antibody or an immunologically active fragment thereof specific for a polypeptide encoded by SEQ ID No. 2 in the preparation of a composition for use in the treatment of breast cancer and breast cancer metastasis.

9. Use of an antisense RNA specific for the nucleic acid having SEQ ID No.2 in the preparation of a composition for use in the treatment of breast cancer and breast cancer metastasis.

## Patentansprüche

1. Verfahren zum Screenen auf Antagonisten des Polypeptids, das durch SEQ.-ID.-Nr. 2 kodiert wird, die zur Inhibierung oder zumindest Verringerung der Aktivität des Polypeptids fähig sind, zur Behandlung von Brustkrebs und Brustkrebsmetastase, das die folgenden Schritte umfasst:
(a) Inkubieren eines Polypeptids, das durch SEQ.-ID.-Nr. 2 kodiert wird, oder eines Fragments davon mit der zu screenenden Verbindung; und
(b) Detektieren der Wechselwirkung zwischen der Verbindung und dem Polypeptid oder seines Fragments.

2. Verfahren nach Anspruch 1, worin das Polypeptidfragment aus der katalytischen Domäne ausgewählt ist.

3. Verwendung eines Antikörpers oder immunologisch aktiven Fragments davon, der/das für ein Polypeptid, das durch SEQ.-ID.-Nr. 2 kodiert wird, spezifisch ist, zur Herstellung eines Diagnostikums für Brustkrebs und Brustkrebsmetastase.

4. Verwendung nach Anspruch 3, worin das Polypeptidfragment aus der katalytischen Domäne ausgewählt ist.

5. Verfahren zum Diagnostizieren von Brustkrebs oder Brustkrebsmetastase, in dem die Expression des Polypeptids, das durch SEQ.-ID.-Nr. 2 kodiert wird, mit einer Nukleinsäure mit der SEQ.-ID.-Nr. 2 oder einem Fragment davon bestimmt wird.

6. Verfahren nach Anspruch 5, worin die Nukleinsäure DNA oder RNA ist.

7. Verfahren nach Anspruch 5 oder 6, worin die Nukleinsäure eine Sequenz, wie in SEQ.-ID.-Nr. I angegeben, hat.

8. Verwendung eines Antikörpers oder eines immunologisch aktiven Fragments davon, der/das für ein Polypeptid, das durch SEQ.-ID.-Nr. 2 kodiert wird, spezifisch ist, bei der Herstellung einer Zusammensetzung zur Verwendung bei der Behandlung von Brustkrebs und Brustkrebsmetastase.

9. Verwendung einer Antisense-RNA, die für die Nukleinsäure mit der SEQ.-ID.-Nr. 2 spezifisch ist, bei der Herstellung einer Zusammensetzung zur Verwendung bei der Behandlung von Brustkrebs und Brustkrebsmetastase.

## Revendications

1. Méthode de criblage d'antagonistes du polypeptide codé par SEQ ID NO : 2 capable d'inhiber ou au moins de diminuer l'activité du polypeptide pour traiter un cancer du sein et une métastase de cancer du sein, comprenant les étapes consistant à :
(a) incuber un polypeptide codé par SEQ ID NO : 2 ou un fragment de celui-ci avec le composé à cribler ; et
(b) détecter l'interaction entre le composé et le polypeptide ou son fragment.

2. Méthode selon la revendication 1, dans laquelle le fragment de polypeptide est choisi dans le domaine catalytique.

3. Utilisation d'un anticorps ou d'un fragment immunologiquement actif de celui-ci spécifique d'un polypeptide codé par SEQ ID NO : 2 pour la fabrication d'un diagnostic de cancer du sein et de métastase de cancer du sein.

4. Utilisation selon la revendication 3, dans laquelle le fragment de polypeptide est choisi dans le domaine catalytique.

5. Méthode de diagnostic de cancer du sein ou de métastase de cancer du sein dans laquelle l'expression du polypeptide codé par SEQ ID NO : 2 est déterminée avec un acide nucléique ayant SEQ ID NO : 2 ou un fragment de celui-ci.

6. Méthode selon la revendication 5, dans laquelle l'acide nucléique est l'ADN ou l'ARN.

7. Méthode selon la revendication 5 ou 6, dans laquelle l'acide nucléique a une séquence comme indiqué dans SEQ ID NO : 1.

8. Utilisation d'un anticorps ou d'un fragment immunologiquement actif de celui-ci spécifique d'un polypeptide codé par SEQ ID NO : 2 dans la préparation d'une composition destinée à être utilisée dans le traitement d'un cancer du sein et d'une métastase de cancer du sein.

9. Utilisation d'un ARN anti-sens spécifique de l'acide nucléique ayant SEQ ID NO : 2 dans la préparation d'une composition destinée à être utilisée dans le traitement d'un cancer du sein et d'une métastase de cancer du sein.
